(19) Europäisches Patentamt
European Patent Office
Office européen des brevets

(11) Veröffentlichungsnummer: **0 042 533**
**B1**

(12) # EUROPÄISCHE PATENTSCHRIFT

(45) Veröffentlichungstag der Patentschrift: **14.03.84**

(51) Int. Cl.³: **C 07 D 317/46**, C 07 D 319/20, A 01 N 47/30, A 01 N 47/34

(21) Anmeldenummer: **81104427.0**

(22) Anmeldetag: **10.06.81**

(54) **Substituierte Benzoyl-(thio)harnstoffe, Verfahren zu ihrer Herstellung und ihre Verwendung als Schädlingsbekämpfungsmittel.**

(30) Priorität: **21.06.80 DE 3023328**

(43) Veröffentlichungstag der Anmeldung: **30.12.81 Patentblatt 81/52**

(45) Bekanntmachung des Hinweises auf die Patenterteilung: **14.03.84 Patentblatt 84/11**

(84) Benannte Vertragsstaaten: **AT BE CH DE FR GB IT LI NL SE**

(56) Entgegenhaltungen:
**EP - A - 0 005 756**
**DE - A - 2 601 780**
**DE - A - 2 637 947**
**DE - A - 2 848 794**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Sirrenberg, Wilhelm, Dr., Wuppertaler Strasse 21, D-4322 Sprockhoevel (DE)**
Erfinder: **Marhold, Albrecht, Dr., Carl-Duisberg-Strasse 329, D-5090 Leverkusen (DE)**
Erfinder: **Hammann, Ingeborg, Dr., Belfortstrasse 9, D-5000 Köln (DE)**
Erfinder: **Krehan, Ingomar, Dr., Ludwig-Jahn-Strasse 54, D-5000 Köln 40 (DE)**
Erfinder: **Stendel, Wilhelm, Dr., In den Birken 55, D-5600 Wuppertal 1 (DE)**

**EP 0 042 533 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Substituierte Benzoyl-(thio)harnstoffe, Verfahren zu ihrer Herstellung und ihre Verwendung als Schädlingsbekämpfungsmittel

Die Erfindung betrifft neue N-Fluoralkylendioxy-phenyl-N'-benzoyl-(thio)harnstoffe, Verfahren zu deren Herstellung und deren Verwendung als Schädlingsbekämpfungsmittel, insbesondere als Insektizide.

Es ist bereits bekanntgeworden, daß bestimmte Benzoylharnstoffe, wie z. B. N-(4-Chlor-phenyl)-N'-(2,6-difluorbenzoyl)-harnstoff, N-(4-Trifluor-methoxy-phenyl)-N'-(2-chlor-benzoyl)-harnstoff und N-(2,2,4,4,-Tetrafluor-1,3-benzdioxin-6-yl)-N'-(2-chlor-benzoyl)-harnstoff, insektizide Eigenschaften aufweisen (vgl. deutsche Offenlegungsschriften 2 123 236, 2 601 780 und 2 637 947).

Es wurden nun neue substituierte N-Fluoralkylendioxy-phenyl-N'-benzoyl-(thio)harnstoffe der Formel I

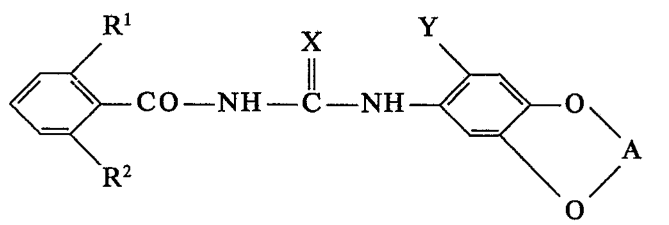

(I)

gefunden, in welcher

$R^1$ für Wasserstoff, Halogen oder Alkyl mit 1 bis 6 Kohlenstoffatomen steht,
$R^2$ für Wasserstoff oder Halogen steht,
X für Sauerstoff oder Schwefel steht,
Y für Wasserstoff, Halogen, Alkyl mit 1 bis 6 Kohlenstoffatomen oder Halogenalkyl mit 1 bis 6 Kohlenstoffatomen steht und
A für durch Fluor und gegebenenfalls zusätzlich durch Chlor substituiertes Alkylen mit 1 oder 2 Kohlenstoffatomen steht.

Man erhält erfindungsgemäß die neuen Verbindungen der Formel (I), wenn man

a) substituierte Benzoyl-iso(thio)cyanate der Formel II

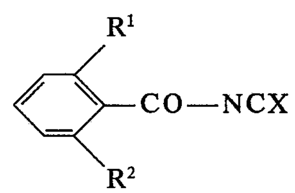

(II)

in welcher

$R^1$, $R^2$ und X die oben angegebenen Bedeutungen haben,

mit Fluoralkylendioxy-anilinen der Formel III

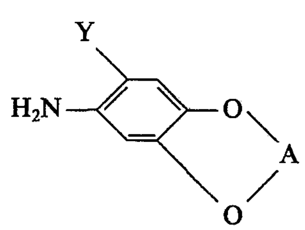

(III)

in welcher

Y und A die oben angegebenen Bedeutungen haben,

gegebenenfalls in Gegenwart eines Verdünnungsmittels umsetzt oder

b) substituierte Benzamide der Formel IV

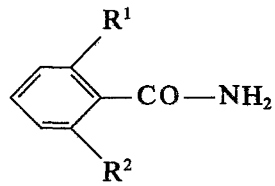

(IV)

in welcher

$R^1$ und $R^2$ die oben angegebenen Bedeutungen haben,

mit Fluoralkylendioxy-phenyl-iso(thio)cyanaten der Formel V

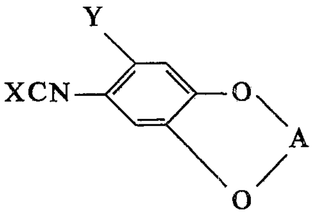

(V)

in welcher

X, Y und A die oben angegebenen Bedeutungen haben,

gegebenenfalls unter Verwendung eines Verdünnungsmittels umsetzt,
und die gebildeten Endprodukte der Formel I isoliert.

Die neuen Verbindungen der Formel (I) verfügen über Eigenschaften, die ihre Verwendung als Schädlingsbekämpfungsmittel ermöglichen, insbesondere zeichnen sie sich durch eine hervorragende insektizide Wirksamkeit aus.

Überraschenderweise zeigen die erfindungsgemäßen N-Fluoralkylendioxy-phenyl-N′-benzoyl-harnstoffe der Formel (I) eine wesentlich höhere insektizide Wirkung als die aus dem Stand der Technik bekannten Verbindungen.

In der Definition von $R^1$ und Y bedeutet Alkyl geradkettiges oder verzweigtes Alkyl mit 1 bis 6, vorzugsweise 1 bis 4 Kohlenstoffatomen. Genannt seien Methyl, Ethyl, n- und i-Propyl, n-, i- und t-Butyl, n-Pentyl und n-Hexyl. Bevorzugt seien Methyl und Ethyl, insbesondere Methyl, genannt.

In Halogenalkyl Y hat die Alkylgruppe die gleiche Bedeutung wie bei Alkyl $R^1$ und Y, wobei die Alkylgruppe durch 1 oder mehrere, vorzugsweise 1 bis 5, insbesondere 1 bis 3 gleiche oder verschiedene Halogenatome substituiert sein kann. Ganz besonders bevorzugt ist die $CF_3$-Gruppe.

Alkylen A enthält 1 oder 2 Kohlenstoffatome. Alkylen A ist durch 1 bis 4, vorzugsweise 2 oder 3 Fluoratome und gegebenenfalls zusätzlich vorzugsweise durch 1 oder 2, insbesondere 1 Chloratom substituiert.

Halogen $R^1$, $R^2$ und Y steht für Fluor, Chlor, Brom oder Jod, vorzugsweise für Fluor, Chlor und Brom, insbesondere für Fluor und Chlor.

In einer besonderen Ausführungsform der Erfindung hat die Definition der Reste in den Verbindungen der Formel (I) die folgende Bedeutung:

$R^1$ steht für Fluor, Chlor, Brom, Jod oder Methyl,
$R^2$ steht für Wasserstoff, Fluor, Chlor, Brom oder Jod,
X steht für Sauerstoff oder Schwefel,
Y steht für Wasserstoff, Chlor, Methyl oder Trifluormethyl und
A steht für durch Fluor und gegebenenfalls durch Chlor substituiertes Alkylen mit bis zu 2 Kohlenstoffatomen.

Hierbei sind besonders bevorzugt Verbindungen der Formel I, in welcher

$R^1$ für Fluor, Chlor, Brom oder Methyl steht,
$R^2$ für Wasserstoff, Fluor oder Chlor steht,
X für Sauerstoff oder Schwefel steht,
Y für Wasserstoff, Chlor, Methyl oder $CF_3$, insbesondere für Wasserstoff steht, und
A für Difluormethylen oder für Ethylen steht,

welches durch 3 oder 4 Fluoratome oder durch 3 Fluoratome und 1 Chloratom substituiert ist.

Verwendet man beispielsweise als Ausgangsstoffe bei Verfahren (a) 2-Fluor-benzoyl-isothiocyanat

und 3,4-(Tetrafluorethylendioxy)-anilin sowie bei Verfahren (b) 2,6-Dibrom-benzamid und 3,4-(Difluormethylendioxy)-phenylisocyanat, so können die entsprechenden Reaktionen durch folgende Formelschemata skizziert werden:

(a)

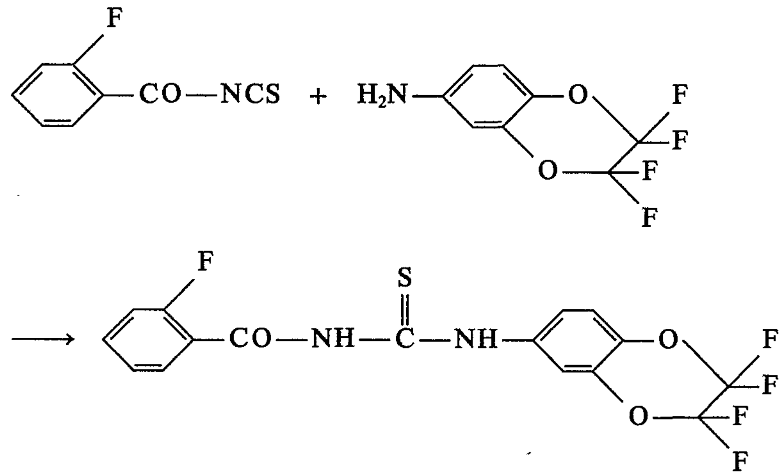

(b)

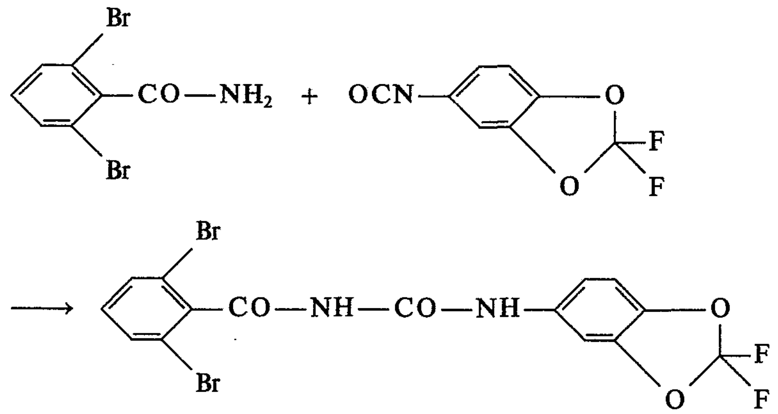

Die bei den Herstellungsverfahren (a) und (b) zu verwendenden Ausgangsstoffe sind durch die Formeln (II) und (III) bzw. (IV) und (V) definiert. Vorzugsweise stehen in diesen Formeln $R^1$, $R^2$, X, Y und A für diejenigen Reste, welche bei der Definition der Reste $R^1$, $R^2$, X, Y und A in Formel (I) genannt wurden.

Die als Ausgangsverbindungen zu verwendenden Benzoesäureamide (IV) und die entsprechenden Benzoyl-iso(thio)-cyanate (II) sind bekannt oder können analog bekannten Verfahren nach allgemein üblichen Methoden hergestellt werden (vgl. zum Beispiel J. Org. Chem. 30 [1965], 4306—4307, und DE-AS 1 215 144).

Als Beispiele seien genannt:

2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoesäureamid;
2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoylisocyanat sowie
2-Fluor-, 2-Chlor-, 2-Brom-, 2-Jod-, 2-Methyl-, 2,6-Difluor-, 2,6-Dichlor-, 2,6-Dibrom- und 2-Chlor-6-fluorbenzoylisothiocyanat.

Die weiterhin als Ausgangsstoffe zu verwendenden Fluoralkylendioxy-aniline (III) und die entsprechenden Fluoralkylendioxy-phenyl-iso(thio)cyanate (V) sind ebenfalls bekannt oder können analog bekannten Verfahren nach allgemein üblichen Methoden hergestellt werden (vgl. deutsche Offenlegungsschrift 2 848 531).

Als Beispiele seien genannt:

5-Amino-, 5-Isocyanato- und
5-Isothiocyanato-2,2-difluor-1,3-benzdioxol,
5-Amino-, 5-Isocyanato- und
5-Isothiocyanato-6-chlor-2,2-difluor-1,3-benzdioxol,
5-Amino-, 5-Isocyanato- und
5-Isothiocyanato-6-methyl-2,2-difluor-1,3-benzdioxol,

5-Amino-, 5-Isocyanato- und
5-Isothiocyanato-6-trifluormethyl-2,2-difluor-1,3-benzdioxol,
6-Amino-, 6-Isocyanato- und
6-Isothiocyanato-2,2-difluor-1,4-benzdioxin,
6-Amino-, 6-Isocyanato- und
6-Isothiocyanato-2,2,3-trifluor-1,4-benzdioxin,
6-Amino-, 6-Isocyanato- und
6-Isothiocyanato-3-chlor-2,2,3-trifluor-1,4-benzdioxin,
6-Amino-, 6-Isocyanato- und
6-Isothiocyanato-7-chlor-2,2,3-trifluor-1,4-benzdioxin,
6-Amino-, 6-Isocyanato- und
6-Isothiocyanato-7-methyl-2,2,3-trifluor-1,4-benzdioxin sowie
6-Amino-, 6-Isocyanato- und
6-Isothiocyanato-7-trifluormethyl-2,2,3-trifluor-1,4-benzdioxin.

Die Verfahrensvarianten zur Herstellung der neuen N-Fluoralkylendioxy-phenyl-N'-benzoyl-(thio)-harnstoffe werden vorzugsweise unter Verwendung von Verdünnungsmitteln durchgeführt.

Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glycoldimethylether und Diglycoldimethylether, Tetrahydrofuran und Dioxan, Ketone, wie Aceton, Methyl-ethyl-, Methylisopropyl- und Methylisobutylketon, Ester, wie Essigsäuremethylester und -ethylester, Nitrile, wie z. B. Acetonitril und Propionitril, Amide, wie z. B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperatur kann innerhalb eines größeren Bereiches variiert werden. Im allgemeinen arbeitet man zwischen 20 und 180°C, vorzugsweise bei 60 bis 120°C. Die erfindungsgemäßen Verfahrensvarianten werden im allgemeinen bei Normaldruck durchgeführt.

Zur Durchführung der erfindungsgemäßen Verfahrensvarianten werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel durchgeführt, und das Reaktionsgemisch wird mehrere Stunden bei der erforderlichen Temperatur gerührt. Danach läßt man das Reaktionsgemisch abkühlen und saugt im Falle, daß die Endprodukte im verwendeten Lösungsmittel schwer löslich sind, vom auskristallisierten Produkt ab. Ansonsten erfolgt die Isolierung und gegebenenfalls Reinigung nach allgemein üblichen Methoden, z. B. durch Abdampfen des Lösungsmittels (gegebenenfalls unter vermindertem Druck). Zur Charakterisierung dient der Schmelzpunkt.

Wie bereits dargelegt, betrifft die vorliegende Erfindung außer den neuen Verbindungen der Formel I und deren Herstellung auch Schädlingsbekämpfungsmittel, welche Verbindungen der Formel I enthalten, die Herstellung dieser Schädlingsbekämpfungsmittel und ihre Verwendung. Die erfindungsgemäßen Verbindungen der Formel I zeigen auch eine fungizide Wirksamkeit, was ihren Wert bei der Verwendung als Schädlingsbekämpfungsmittel im Pflanzenschutz steigert.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, ganz besonders bevorzugt zur Bekämpfung von Insekten, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B.
Oniscus asellus, Armadillidium vulgare, Porcellio scaber.
Aus der Ordnung der Diplopoda z. B. Blaniulus guttulatus.
Aus der Ordnung der Chilopoda z. B. Geophilus carpophagus, Scutigera spec.
Aus der Ordnung der Symphyla z. B. Scutigerella immaculata.
Aus der Ordnung der Thysanura z. B. Lepisma saccharina.
Aus der Ordnung der Collembola z. B. Onychiurus armatus.
Aus der Ordnung der Orthoptera z. B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung der Dermaptera z. B. Forficula auricularia.
Aus der Ordnung der Isoptera z. B. Reticulitermes spp.
Aus der Ordnung der Anoplura z. B. Phylloera vastatrix, Pemphigus spp., Pediculus humanus corporis, Haematopinus spp., Linognathus spp.
Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicornyne brassicae, Cryptomyzus ribis, Doralis fabae, Doralis pomi, Eriosoma lanigerum, Hyalopterus arundinis, Macrosiphium avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella auroantii, Aspidiotus hederae, Pseudococcus spp., Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris sp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthestische Stoffe, Feinstverkapselungen in polymeren Stoffen und in Hüllmassen für Saatgut, ferner in Formulierungen mit Brennsätzen wie Räucherpatronen, -dosen und -spiralen sowie ULV-Kalt- und -Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe und durch Mikroorganismen hergestellte Stoffe.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten vorzugsweise von ektoparasitierenden Insekten auf dem veterinärmedizinischen Gebiet bzw. auf dem Gebiet der Tierhaltung.

Die Anwendung der erfindungsgemäßen Wirkstoffe geschieht hier in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z. B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck der Insekten abhängig und lassen sich nach den üblichen Methoden leicht ermitteln und festlegen. Die neuen Verbindungen können bei Rindern z. B. in Dosiermengen von 0,01 bis 1 mg/kg Körpergewicht eingesetzt werden.

Die insektizide Wirksamkeit der neuen erfindungsgemäßen Verbindungen ergibt sich aus den folgenden Beispielen:

## Beispiel A

### Plutella-Test

| | |
|---|---|
| Lösungsmittel: | 3 Gewichtsteile Dimethylformamid |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z. B. bei einem Versuch mit einer Wirkstoffkonzentration von 0,001% die Verbindungen Nr. 1, 2, 11, 12, 27, 32 bis 38, 49, 53, 56 bis 59 und 63 einen Abtötungsgrad von 100% nach 7 Tagen.

## Beispiel B

### Laphygma-Test

Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen des Eulenfalters (Laphygma frugiperda) besetzt, solange die Blätter noch feucht sind.

Nach der gewünschten Zeit wird die Abtötung in % bestimmt. Dabei beutet 100%, daß alle Raupen abgetötet wurden; 0% bedeutet, daß keine Raupen abgetötet wurden.

Bei diesem Test zeigen z. B. bei einem Versuch mit einer Wirkstoffkonzentration von 0,001% die folgenden Verbindungen Nr. 1, 2, 6, 7, 10 bis 12, 20, 21, 27, 31, 33, 35 bis 39, 44, 49 und 57 bis 59 einen Abtötungsgrad von 100% nach 7 Tagen.

## Beispiel C

### Mückenlarven-Test

Testtiere: Aedes aegypti
Lösungsmittel: Aceton, 99 Gewichtsteile
Emulgator: Benzylhydroxidiphenylglykolether, 1 Gewichtsteil

Zur Herstellung einer zweckmäßigen Wirkstoffzubereitung löst man 2 Gew.-Teile Wirkstoff in 1000 Volumenteilen Lösungsmittel, das Emulgator in der oben angegebenen Menge enthält. Die so erhaltene Lösung wird mit Wasser auf die gewünschten geringeren Konzentrationen verdünnt.

Man füllt die wäßrigen Wirkstoffzubereitungen der gewünschten Konzentration in Gläser und setzt anschließend etwa 25 Mückenlarven in jedes Glas ein.

Nach 21 Tagen wird der Abtötungsgrad in % bestimmt. Dabei bedeutet 100%, daß alle Larven abgetötet worden sind. 0% bedeutet, daß überhaupt keine Larven abgetötet worden sind.

Bei diesem Test zeigen z. B. bei einem Versuch mit Verdünnungen von $10^{-3}$ bis $10^{-4}$ ppm die Verbindungen Nr. 1, 36, 39 und 59 einen Abtötungsgrad von 100% in 21 Tagen.

## Beispiel D

### Test mit Lucilia cuprina res.-Larven

Lösungsmittel: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z. B. bei einem Versuch mit einer Wirkstoffkonzentration von 100 ppm die Verbindungen Nr. 6, 10 bis 12, 28, 31, 33 bis 35, 54, 55, 57 und 59 Abtötungsgrade zwischen 50 und 100%.

Die Herstellung der erfindungsgemäßen Verbindungen soll durch die folgenden Beispiele erläutert werden:

Beispiel 1

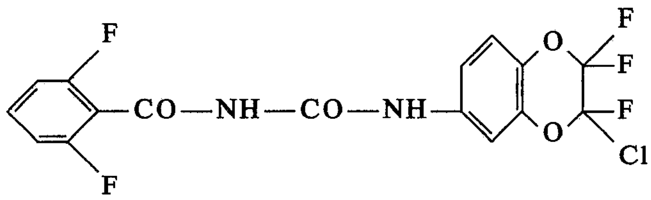

(1)

Zu einer Lösung von 4,8 g (0,02 Mol) 6-Amino-2,2,3-trifluor-3-chlor-benzdioxin-(1,4) in 40 ml Toluol fügt man bei 60° C 3,66 g (0,02 Mol) 2,6-Difluorbenzoylisocyanat in 20 ml Toluol. Der Ansatz wird eine Stunde bei 80° C gerührt und wird dann auf Raumtemperatur abgekühlt. Die ausgefallene Substanz wird abgesaugt, mit wenig Toluol gewaschen und getrocknet. Man erhält 8,3 g (98% der Theorie) mit einem Schmelzpunkt von 197° C.

Beispiel 2

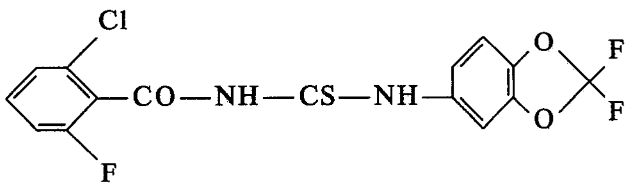

(2)

Zu einer Lösung von 3,46 g (0,02 Mol) 5-Amino-2,2-difluorbenzdioxol in 40 ml Toluol fügt man bei 60° C eine Lösung von 4,3 g (0,02 Mol) 2-Chlor-6-fluor-benzoyl-isothiocyanat in 10 ml Toluol und rührt den Ansatz eine Stunde bei 80° C. Danach kühlt man auf Raumtemperatur und trennt die ausgefallene Substanz ab. Man erhält nach dem Trocknen 7,4 g (95% der Theorie) Substanz mit einem Schmelzpunkt von 174° C.

Beispiel 3

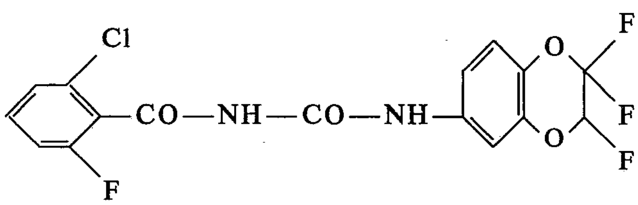

(3)

Zu einer 100° C warmen Lösung von 5,22 g (0,03 Mol) 2-Chlor-6-fluor-benzamid in 80 ml Toluol gibt man 6,93 g (0,03 Mol) 6-Isocyanato-2,2,3-trifluor-benzdioxin-(1,4) und rührt den Ansatz 28 Stunden bei 100° C. Beim Abkühlen auf Raumtemperatur fällt die Substanz aus. Sie wird abgesaugt und getrocknet und zeigt einen Schmelzpunkt von 194° C. Ausbeute 7,5 g (61,5% der Theorie). Die Verbindung ist identisch mit einer nach Verfahren (a) hergestellten Probe.

In analoger Arbeitsweise werden gemäß den Beispielen 1 bis 3 auch die in den nachstehenden Tabellen aufgeführten Verbindungen hergestellt, wobei die Verfahrensvarianten (a) und (b) in gleicher Weise eingesetzt werden.

Tabelle I

Verbindungen der Formel Ia

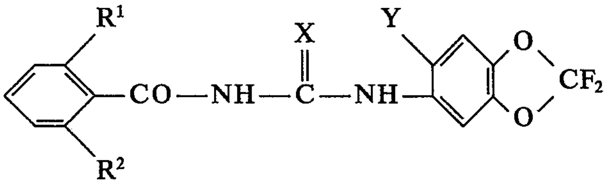

(Ia)

| Produkt Nr. | $R^1$ | $R^2$ | Y | X | Schmelzpunkt °C |
|---|---|---|---|---|---|
| 4 | H | H | H | O | 217 |
| 5 | F | H | H | O | 167 |
| 6 | Cl | H | H | O | 202 |
| 7 | Br | H | H | O | 201 |
| 8 | J | H | H | O | 196 |
| 9 | $CH_3$ | H | H | O | 200 |
| 10 | Cl | Cl | H | O | 205 |
| 11 | Cl | F | H | O | 221 |
| 12 | F | F | H | O | 220 |
| 13 | Cl | H | $CF_3$ | O | 156 |
| 14 | Cl | F | $CF_3$ | O | 190 |
| 15 | F | F | $CF_3$ | O | 181 |
| 16 | Cl | H | Cl | O | 215 |
| 17 | Br | H | Cl | O | 213 |
| 18 | Cl | Cl | Cl | O | 265 |
| 19 | Cl | F | Cl | O | 247 |
| 20 | F | F | Cl | O | 228 |
| 21 | Cl | H | H | S | 158 |
| 22 | Cl | H | $CF_3$ | S | 172 |
| 23 | Cl | H | Cl | S | 133 |
| 24 | Cl | Cl | Cl | S | 197 |
| 25 | Cl | F | Cl | S | 174 |
| 26 | F | F | Cl | S | 156 |
| 27 | F | F | H | S | |

Tabelle II

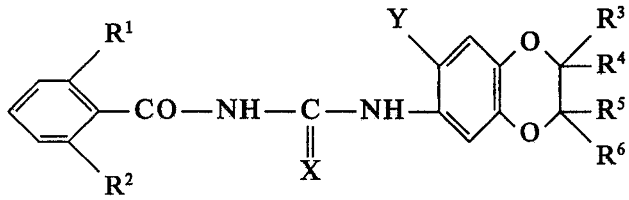

(Ib)

| Produkt Nr. | $R^1$ | $R^2$ | $R^3$ | $R^4$ | $R^5$ | $R^6$ | Y | X | Schmelzpunkt °C |
|---|---|---|---|---|---|---|---|---|---|
| 28 | Cl | H | F | F | H | H | H | O | 183 |
| 29 | Br | H | F | F | H | H | H | O | 177 |
| 30 | Cl | Cl | F | F | H | H | H | O | 227 |
| 31 | Cl | F | F | F | H | H | H | O | 201 |
| 32 | F | F | F | F | H | H | H | O | 214 |
| 33 | Cl | H | F | F | F | H | H | O | 186 |
| 34 | Br | H | F | F | F | H | H | O | 170 |
| 35 | Cl | F | F | F | F | H | H | O | 198 |
| 36 | Cl | H | F | F | F | Cl | H | O | 184 |
| 37 | Br | H | F | F | F | Cl | H | O | 183 |
| 38 | $CH_3$ | H | F | F | F | Cl | H | O | 164 |
| 39 | Cl | Cl | F | F | F | Cl | H | O | 210 |
| 40 | F | H | F | H | F | F | $CH_3$ | O | 203 |
| 41 | Cl | H | F | H | F | F | $CH_3$ | O | 148 |
| 42 | Cl | Cl | F | H | F | F | $CH_3$ | O | 215 |
| 43 | Cl | F | F | H | F | F | $CH_3$ | O | 169 |
| 44 | F | F | F | H | F | F | $CH_3$ | O | 198 |
| 45 | F | H | F | H | F | F | Cl | O | 168 |
| 46 | Br | H | F | H | F | F | Cl | O | 180 |
| 47 | $CH_3$ | H | F | H | F | F | Cl | O | 187 |
| 48 | Cl | Cl | F | H | F | F | Cl | O | 231 |
| 49 | F | F | F | H | F | F | Cl | O | 206 |
| 50 | Cl | H | F | F | H | H | H | S | 128 |
| 51 | Cl | Cl | F | F | H | H | H | S | 189 |
| 52 | Cl | F | F | F | H | H | H | S | 158 |
| 53 | F | F | F | F | H | H | H | S | 137 |
| 54 | Cl | H | F | F | F | H | H | S | 116 |
| 55 | Cl | Cl | F | F | F | H | H | S | 158 |
| 56 | Cl | F | F | F | F | H | H | S | 146 |
| 57 | Cl | H | F | F | F | Cl | H | S | 148 |
| 58 | Cl | F | F | F | F | Cl | H | S | 193 |
| 59 | F | F | F | F | F | Cl | H | S | 175 |

Fortsetzung

| Produkt Nr. | $R^1$ | $R^2$ | $R^3$ | $R^4$ | $R^5$ | $R^6$ | Y | X | Schmelzpunkt °C |
|---|---|---|---|---|---|---|---|---|---|
| 60 | Cl | H | F | H | F | F | $CH_3$ | S | 173 |
| 61 | Cl | F | F | H | F | F | $CH_3$ | S | 205 |
| 62 | Cl | Cl | F | H | F | F | Cl | S | 202 |
| 63 | F | F | F | H | F | F | Cl | S | 186 |

**Patentansprüche**

1. Verbindungen der allgemeinen Formel I

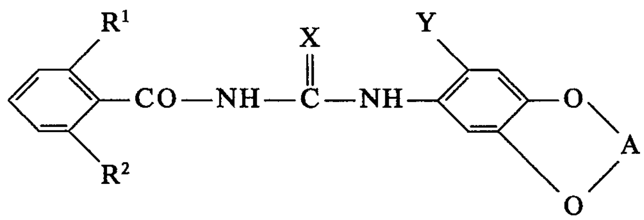

(I)

in welcher

$R^1$ für Wasserstoff, Halogen oder Alkyl mit 1 bis 6 Kohlenstoffatomen steht,
$R^2$ für Wasserstoff oder Halogen steht,
X für Sauerstoff oder Schwefel steht,
Y für Wasserstoff, Halogen, Alkyl mit 1 bis 6 Kohlenstoffatomen oder Halogenalkyl mit 1 bis 6 Kohlenstoffatomen steht und
A für durch Fluor und gegebenenfalls zusätzlich durch Chlor substituiertes Alkylen mit 1 oder 2 Kohlenstoffatomen steht.

2. Verbindungen gemäß Anspruch 1, in welchem

$R^1$ für Wasserstoff, Halogen oder Alkyl mit 1 bis 6 Kohlenstoffatomen steht,
$R^2$ für Wasserstoff oder Halogen steht,
X für Sauerstoff oder Schwefel steht,
Y für Wasserstoff, Halogen, Alkyl mit 1 bis 6 Kohlenstoffatomen oder Halogenalkyl mit 1 bis 6 Kohlenstoffatomen und 1 bis 5 Halogenatomen steht und
A für durch Fluor und gegebenenfalls zusätzlich durch Chlor substituiertes Alkylen mit 1 oder 2 Kohlenstoffatomen steht.

3. Verbindungen gemäß den Ansprüchen 1 oder 2, in welchen

$R^1$ für Fluor, Chlor, Brom, Jod oder Methyl steht,
$R^2$ für Wasserstoff, Fluor, Chlor, Brom oder Jod steht,
X für Sauerstoff oder Schwefel steht,
Y für Wasserstoff, Chlor, Methyl oder Trifluormethyl steht und
A für durch Fluor und gegebenenfalls durch Chlor substituiertes Alkylen mit bis zu 2 Kohlenstoffatomen steht.

4. Verbindungen gemäß Ansprüchen 1 bis 3, in welchen

$R^1$ für Fluor, Chlor, Brom oder Methyl steht,
$R^2$ für Wasserstoff, Fluor oder Chlor steht,
X für Sauerstoff oder Schwefel steht,
Y für Wasserstoff, Chlor, Methyl oder $CF_3$ steht und
A für Difluormethylen oder für Ethylen steht, welches durch 3 oder 4 Fluoratome oder durch 3 Fluoratome und 1 Chloratom substituiert ist.

5. Verfahren zur Herstellung der Verbindungen gemäß den Ansprüchen 1 bis 4, dadurch gekennzeichnet, daß man

(a) substituierte Benzoyl-iso(thio)cyanate der Formel II

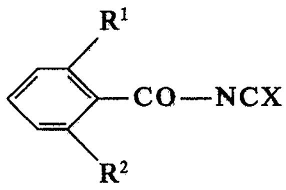

(II)

in welcher

$R^1$, $R^2$ und X die oben angegebenen Bedeutungen haben,

mit Fluoralkylendioxy-anilinen der Formel III

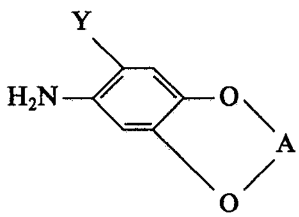

(III)

in welcher

Y und A die oben angegebenen Bedeutungen haben,

gegebenenfalls in Gegenwart eines Verdünnungsmittels umsetzt oder

(b) substituierte Benzamide der Formel IV

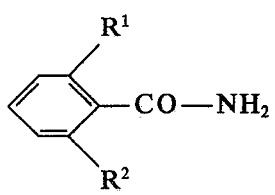

(IV)

in welcher

$R^1$ und $R^2$ die oben angegebenen Bedeutungen haben,

mit Fluoralkylendioxy-phenyl-iso(thio)cyanaten der Formel V

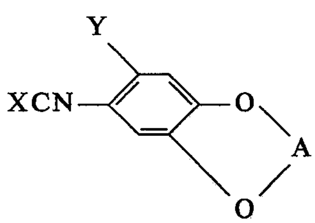

(V)

in welcher

X, Y und A die oben angegebenen Bedeutungen haben,

gegebenenfalls unter Verwendung eines Verdünnungsmittels umsetzt und die gebildeten Endprodukte der Formel I isoliert.

6. Schädlingsbekämpfungsmittel, gekennzeichnet durch den Gehalt an mindestens einer Verbindung gemäß den Ansprüchen 1 bis 4.

7. Herstellung der Schädlingsbekämpfungsmittel gemäß Anspruch 6, dadurch gekennzeichnet, daß man mindestens eine Verbindung gemäß den Ansprüchen 1 bis 4 mit inerten Streck- und

Verdünnungsmitteln und gegebenenfalls mit oberflächenaktiven Stoffen mischt.

8. Verwendung der Verbindungen der Ansprüche 1 bis 4 zur Bekämpfung von Insekten, die in der Landwirtschaft, in Forsten, im Vorrats- und Materialschutz sowie auf dem Hygienesektor vorkommen.

9. Verwendung der Schädlingsbekämpfungsmittel gemäß Anspruch 6 zur Bekämpfung von Insekten, die in der Landwirtschaft, in Forsten, im Vorrats- und Materialschutz sowie auf dem Hygienesektor vorkommen.

10. Verbindungen der allgemeinen Formel I

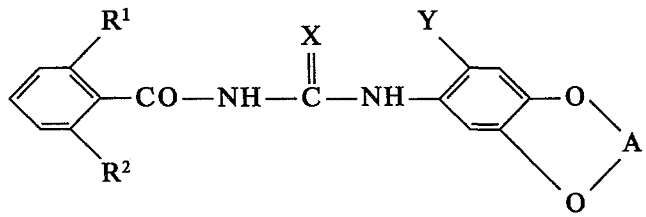

(I)

in welcher

$R^1$, $R^2$, X, Y und A die in Anspruch 1 angegebenen Bedeutungen haben,

zur Bekämpfung von Parasiten.

**Claims**

1. Compounds of the general formula I

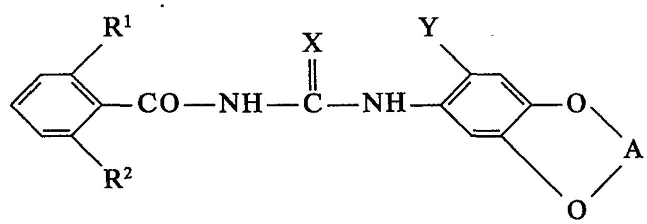

(I)

in which

$R^1$ represents hydrogen, halogen or alkyl with 1 to 6 carbon atoms,
$R^2$ represents hydrogen or halogen,
X represents oxygen or sulphur,
Y represents hydrogen, halogen, alkyl with 1 to 6 carbon atoms, or halogenoalkyl with 1 to 6 carbon atoms, and
A represents alkylene which has 1 or 2 carbon atoms which is substituted by fluorine and optionally additionally substituted by chlorine.

2. Compounds according to Claim 1, in which

$R^1$ represents hydrogen, halogen or alkyl with 1 to 6 carbon atoms,
$R^2$ represents hydrogen or halogen,
X represents oxygen or sulphur,
Y represents hydrogen, halogen, alkyl with 1 to 6 carbon atoms or halogenoalkyl with 1 to 6 carbon atoms and 1 to 5 halogen atoms and
A represents alkylene which has 1 or 2 carbon atoms and is substituted by fluorine and optionally additionally substituted by chlorine.

3. Compounds according to Claims 1 or 2, in which

$R^1$ represents fluorine, chlorine, bromine, iodine or methyl,
$R^2$ represents hydrogen, fluorine, chlorine, bromine or iodine,
X represents oxygen or sulphur,
Y represents hydrogen, chlorine, methyl or trifluoromethyl and
A represents alkylene which has up to 2 carbon atoms and is substituted by fluorine and optionally substituted by chlorine.

4. Compounds according to Claims 1 to 3, in which

$R^1$ represents fluorine, chlorine, bromine or methyl,
$R^2$ represents hydrogen, fluorine or chlorine,
X represents oxygen or sulphur,
Y represents hydrogen, chlorine, methyl or $CF_3$ and
A represents difluoromethylene, or ethylene which is substituted by 3 or 4 fluorine atoms or by 3 fluorine atoms and 1 chlorine atom.

5. Process for the preparation of the compounds according to Claims 1 to 4, characterised in that (a) substituted benzoyl iso(thio)cyanates of the formula II

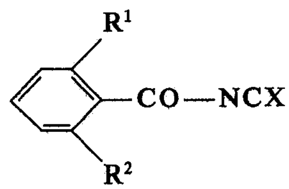
(II)

in which

$R^1$, $R^2$ and X have the abovementioned meanings,

are reacted with fluoralkylenedioxy-anilines of the formula III

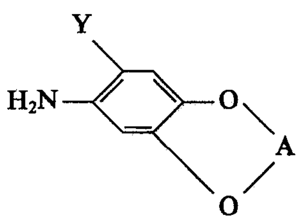
(III)

in which

Y and A have the abovementioned meanings,

if appropriate in the presence of a diluent, or (b) substituted benzamides of the formula IV

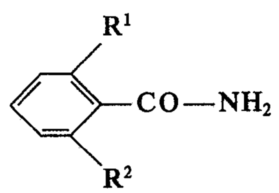
(IV)

in which

$R^1$ and $R^2$ have the abovementioned meanings,

are reacted with fluoralkylenedioxy-phenyl iso(thio)cyanates of the formula V

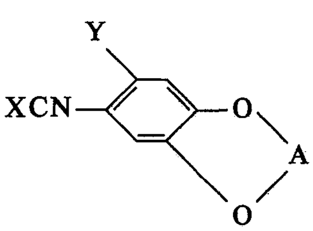
(V)

in which

X, Y and A have the abovementioned meanings,

if appropriate using a diluent, and the end products of the formula I are isolated.

6. Pest-combating agents, characterised in that they contain at least one compound according to Claims 1 to 4.

7. Preparation of the pest-combating agents according to Claim 6, characterised in that at least one compound according to Claims 1 to 4 is mixed with inert extenders and diluents and, if appropriate, with surface-active substances.

8. Use of the compounds of Claims 1 to 4 in combating insects, which are encountered in agriculture, in forestry, in the protection of stored products and of materials, and in the hygiene field.

9. Use of the pest-combating agents according to Claim 6, for combating insects, which are encountered in agriculture, in forestry, in the protection of stored products and of materials, and in the hygiene field.

10. Compounds of the general formula I

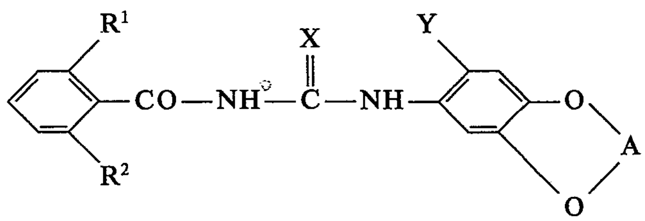

(I)

in which

$R^1$, $R^2$, X, Y and A have the meanings given in Claim 1,

for combating parasites.

**Revendications**

1. Composés de formule générale I

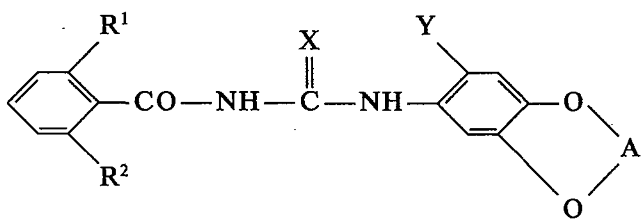

(I)

dans laquelle

- $R^1$ représente l'hydrogène, un halogène ou un groupe alkyle ayant 1 à 6 atomes de carbone,
- $R^2$ est l'hydrogène ou un halogène,
- X est l'oxygène ou le soufre,
- Y est l'hydrogène, un halogène, un groupe alkyle ayant 1 à 6 atomes de carbone ou un groupe halogénalkyle ayant 1 à 6 atomes de carbone et
- A représente un groupe alkylène ayant 1 ou 2 atomes de carbone, substitué par du fluor et aussi, le cas échéant, par du chlore.

2. Composés suivant la revendication 1, dans lesquels

- $R^1$ représente l'hydrogène, un halogène ou un groupe alkyle ayant 1 à 6 atomes de carbone,
- $R^2$ est l'hydrogène ou un halogène,
- X est l'oxygène ou le soufre,
- Y est l'hydrogène, un halogène, un groupe alkyle ayant 1 à 6 atomes de carbone ou un groupe halogénalkyle ayant 1 à 6 atomes de carbone et 1 à 5 atomes d'halogènes et
- A représente un groupe alkylène ayant 1 ou 2 atomes de carbone substitué par du fluor et aussi, le cas échéant, par du chlore.

3. Composés suivant les revendications 1 ou 2, dans lesquels

- $R^1$ représente le fluor, le chlore, le brome, l'iode ou un groupe méthyle,
- $R^2$ est l'hydrogène, le fluor, le chlore, le brome ou l'iode,
- X est l'oxygène ou le soufre,
- Y est l'oxygène, le chlore, le groupe méthyle ou le groupe trifluorométhyle, et

A représente un groupe alkylène ayant jusqu'à 2 atomes de carbone, substitué par du fluor et, le cas échéant, par du chlore.

4. Composés suivant les revendications 1 à 3, dans lesquels

$R^1$ représente du fluor, du chlore, du brome ou le groupe méthyle,
$R^2$ représente de l'hydrogène, du fluor ou du chlore,
X est de l'oxygène ou du soufre,
Y est de l'hydrogène, du chlore, le groupe méthyle ou le groupe $CF_3$, et
A est le groupe difluorméthylène ou un groupe éthylène qui est substitué par 3 ou 4 atomes de fluor ou par 3 atomes de fluor et 1 atome de chlore.

5. Procédé de production des composés suivant les revendications 1 à 4, caractérisé en ce qu'on fait réagir

(a) des benzoyliso(thio)cyanates substitués de formule II

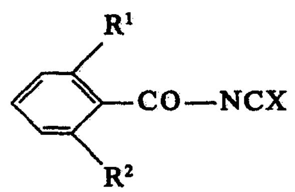

(II)

dans laquelle

$R^1$, $R^2$ et X ont les définitions indiquées ci-dessus,

avec des fluoralkylènedioxyanilines de formule III

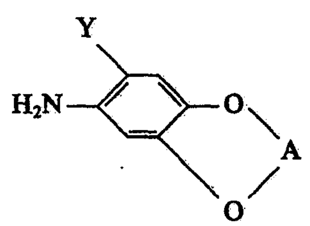

(III)

dans laquelle

Y et A ont les définitions données ci-dessus, éventuellement en présence d'un diluant ou

(b) des benzamides substitués de formule IV

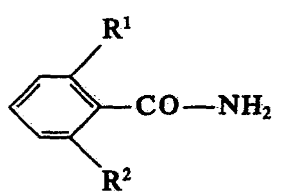

(IV)

dans laquelle

$R^1$ et $R^2$ ont les définitions indiquées ci-dessus,

avec des iso(thio)cyanates de fluoralkylènedioxy-phényle de formule V

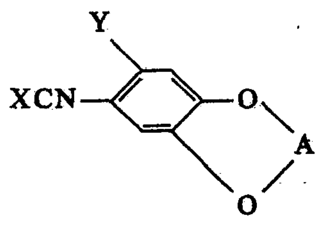

(V)

dans laquelle

X, Y et A ont les définitions données ci-dessus, en utilisant éventuellement un diluant, et on isole les produits finals formés, de formule I.

6. Composition pesticide, caractérisée par le fait qu'elle contient au moins un composé suivant les revendications 1 à 4.

7. Préparation des compositions pesticides suivant la revendication 6, caractérisée en ce qu'on mélange au moins un composé suivant les revendications 1 à 4 avec des diluants inertes et, le cas échéant, avec des agents tensio-actifs.

8. Utilisation des composés suivant les revendications 1 à 4 pour combattre des insectes, rencontrés en agriculture, dans les forêts, dans la protection des denrées et autres produits emmagasinés de même que dans le secteur de l'hygiène.

9. Utilisation des compositions pesticides suivant la revendication 6 pour la lutte contre des insectes, rencontrés en agriculture, dans les forêts, dans la protection des denrées et autres produits emmagasinés de même que dans le secteur de l'hygiène.

10. Composés de formule générale I

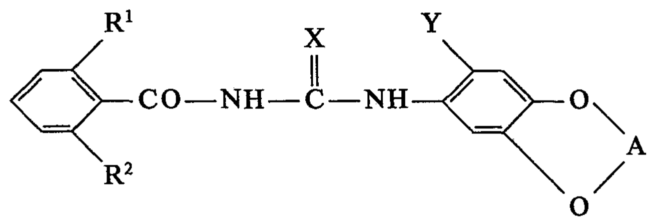

(I)

dans laquelle

$R^1$, $R^2$, X, Y et A ont les définitions indiquées dans la revendication 1.